# EUROPÄISCHE PATENTSCHRIFT

(11) **EP 1 263 426 B1**
(45) Veröffentlichungstag und Bekanntmachung des Hinweises auf die Patenterteilung: **15.03.2006**
(21) Anmeldenummer: 00991166.0
(22) Anmeldetag: 12.12.2000
(51) Int. Cl.: A61K 31/17, A61P 31/10

(54) **ZUBEREITUNGEN ZUR ATRAUMATISCHEN NAGELENTFERNUNG**
PREPARATIONS FOR THE NON-TRAUMATIC EXCISION OF A NAIL
PREPARATIONS POUR ENLEVER DE FACON ATRAUMATIQUE DES ZONES D'ONGLES

(30) Priorität: 03.01.2000 DE 10000053
(43) Veröffentlichungstag der Anmeldung: 11.12.2002
(73) Patentinhaber: Kraemer, Karl, 63225 Langen (DE); Bohn, Manfred, 65719 Hofheim (DE)
(72) Erfinder: Kraemer, Karl, 63225 Langen (DE); Bohn, Manfred, 65719 Hofheim (DE)
(74) Vertreter: Schweitzer, Klaus
(86) Internationale Anmeldenummer: PCT/EP2000/012553
(87) Internationale Veröffentlichungsnummer: WO 2001/049283

(56) Entgegenhaltungen:
- DE-A- 4 337 945
- US-A- 4 402 935
- PATENT ABSTRACTS OF JAPAN vol. 008, no. 106 (C-223), 18. Mai 1984 (1984-05-18) & JP 59 020217 A (KAWAKEN FINE CHEMICAL KK), 1. Februar 1984 (1984-02-01)
- PATENT ABSTRACTS OF JAPAN vol. 1997, no. 03, 31. März 1997 (1997-03-31) & JP 08 291057 A (YUUTOKU YAKUHIN KOGYO KK), 5. November 1996 (1996-11-05)
- PATENT ABSTRACTS OF JAPAN vol. 1996, no. 03, 29. März 1996 (1996-03-29) & JP 07 291856 A (YUUTOKU YAKUHIN KOGYO KK), 7. November 1995 (1995-11-07)

## Beschreibung

Pilzerkrankungen der Fuß- oder Fingernägel (Onychomykosen) sind ein weit verbreitetes Krankheitsbild. Ihre Behandlung ist arterkanntermaßen schwierig und langwierig. Nach in der medizinischen Fachliteratur publizierten Schätzungen haben bis zu 8% der Bevölkerung eine Nagelmykose, in der Altersklasse von 40 bis 60 Jahren sogar bis zu 20%.

Entgegen der bislang vertretenen Meinung handelt es sich bei Nagelmykosen um weit mehr als nur ein kosmetisches Problem. Während erkrankte Zehennägel unter anderem die Fortbewegung unangenehm beeinträchtigen können, führen befallene Fingernägel häufig zu einer negativen Beeinflussung des Selbstwertgefühls und der Lebensqualität. Ein pilzbefallener Nagel kann darüber hinaus auch als Erregerreservoir fungieren und Infektionen an anderen Körperarealen auslösen.

Zur Behandlung von Nagelpilzerkrankungen werden heute verschiedene Wege beschritten:

Eine Behandlungsmethode, die systemische, besteht darin, dass man pilzhemmende Mittel oral verabreicht. Dies kann erfahrungsgemäß bisweilen zu gravierenden, unerwünschten, unter Umständen lebensbedrohenden, Arzneimittelnebenwirkungen führen, da der Wirkstoff über den Blutkreislauf zu dem Infektionsherd gelangen muss.

Eine neuere Methode ist aus toxikologischer Sicht weitaus vorteilhafter und besteht darin, dass man die befallenen Nägel topisch mit speziellen antimykotikahaltigen Nagelzubereitungen, insbesondere mit Lackzubereitungen, behandelt (US 4,957,730; US 5,264,206) . Im Gegensatz zur systemischen Therapie sind bei der topischen Behandlung unerwünschte Nebenwirkungen oder Interaktionen mit anderen systemisch verabreichten Arzneistoffen nahezu ausgeschlossen.

Als zusätzliche lokale Maßnahme zur Verkürzung der Behandlungszeit wird sowohl bei der systemischen als auch bei der lokalen Behandlung von Onychomykosen mit Erfolg die Entfernung der befallenen Nagelareale praktiziert. So wird beispielsweise empfohlen, vor Beginn der eigentlichen Behandlung soviel wie möglich von dem zerstörten Nagelmaterial mit einer Nagelfe le oder einer Schere zu entfernen. Eine Totalextraktion der Nägel, insbesondere bei multiplem Befall, ist für die Patienten in den meisten Fällen nicht zumutbar und birgt das Risiko einer irreversiblen Verletzung der Nagelmatrix mit der Konsequenz des Herauswachsens deformierter Nägel.

Beide Verfahren haben somit schwerwiegende Nachteile. Während das Feilen zwar von den Patienten selbst durchgeführt werden kann, aber im starken Maße zur Verbreitung der Erreger beiträgt, muss das Schneiden mit der Schere oder dem Skalpell aus Sicherheitsgründen von einem erfahrenen Fachmann vorgenommen werden. Insbesondere bei einer großen Gruppe von Problempatienten wie Diabetikern können bei Verletzungen Infektionen die Folge sein, die im schlimmsten Fall Auslöser für Gliedamputationen sein können. Dies ist jedoch sowohl unter ethischen als auch pharmakoökonomischen Gesichtspunkten von großem Nachteil.

Um das damit einhergehende Risiko zu minimieren hat daher zur nagelablösenden Behandlung von pilzbefallenen Nagelarealen an Händen und Füßen eine weitere Methode Eingang in die Therapie gefunden. Dieses Verfahren beruht auf der keratolytischen Wirkung von hochkonzentriertem Harnstoff (40%) bzw. von Kaliumjodid oder Natriumjodid (50%) auf die kranke Nagelsubstanz. Gesundes Nagelmaterial wird dabei nicht angegriffen.

So werden beispielsweise harnstoffhaltige bzw. KJ- oder NaJ-haltige, wasserfreie Pasten auf die erkrankten Nägel aufgetragen bis die gesamte Nageloberfläche dünn bedeckt ist. Die befallenen Finger- und Fußnägel werden dann mit einem Pflaster bzw. einem Verband für jeweils 24 Stunden zugeklebt. Danach wird das Pflaster abgelöst und die betroffenen Finger oder Zehen etwa 10 Minuten in warmen Wasser gebadet. Die Behandlung muss solange fortgesetzt werden bis die aufgeweichte kranke Nagelsubstanz mit einem Schaber völlig entfernt werden kann. Dazu sind im allgemeinen, je nach Ausmaß der Erkrankung und Nageldicke, 7 Tage bis 14 Tage erforderlich. Zum Schutz der den Nagel umgebenden Hautflächen wird das Auftragen von Zinkpaste empfohlen.

Ein durchschlagender Erfolg blieb auch dieser Methode versagt, weil die Behandlung - beispielsweise wegen der störenden und unschön aussehenden Pflaster bzw. Verbände an Zehen und Fingern und der täglich erforderlichen Maßnahmen - von den Patienten vielfach aus kosmetischen sowie Zeitgründen nicht durchgehalten wird. Als nachteilig wird ferner die beim Abnehmen der Pflasterverbände wahrzunehmende sehr unangenehme Geruchsentwicklung angesehen.

Als weiterer Stand der Technik betreffend die Verwendung von harnstoffhaltigen Zubereitungen in Medizin und Kosmetik wurden folgende Dokumente recherchiert: .
Patent Abstracts of Japan, 008(106), C-223, 18/5/1984 und JP 59 020217 A offenbart harnstoffhaltige, wässrige Zubereitungen für die Kosmetik, die unter anderem auch Carboxyvinylpolymer enthalten.
Patent Abstracts of Japan, 1997(3), 31/3/1997 und JP 08 291057 A offenbart entzündungshemmende, analgetisch wirksame harnstoffhaltige Zubereitungen, die Alkohol, Wasser, Pyrrolidoncarboxylsäure und ein Salz der Milchsäure enthalten.
Patent Abstracts of Japan, 1996(3), 29/3/1996 und JP 07 291856 offenbart eine therapeutische, harnstoffhaltige Emulsion zur Behandlung von Dermatosen.
DE-A-43 37 945 offenbart harnstoffhaltige Zubereitungen in Form von Pflastern zur Behandlung von Nagelmykosen. In diesem Nagelpflaster ist ein antimykotischer Wirkstoff enthalten.
US-A-4,402,935 offenbart einen Nagellack zur Verbesserung der Feuchtigkeitsbedingungen in Finger- und Fußnägeln. Eine solche Zubereitung enthält z.B. ein Polyvinylbutyralharz, Wasser und Hamstoff.
US-A-5,993,790 beschreibt die Verwendung eines wasser- und harnstoffhaltigen Nagellacks zur Entfernung von pilzbefallenen Finger- und Fußnägeln. Diese Zubereitung enthält keinen antimykotischen Wirkstoff. Vielmehr wird hier die getrennte und nachfolgende Anwendung einer antimykotisch wirksamen Zubereitung empfohlen.
DE 42 12 105 A1 beschreibt weitgehend wasserfreie Nagellacke, die einen Filmbildner, eine antimykotisch wirksame Substanz und Harnstoff enthalten, zur Behandlung von Onychomykosen. Die gleichzeitige Entfernung und antimykotische Behandlung der pilzbefallenen Nagelareale wird nicht beschrieben.

Die Erfindung bezweckt, eine Formulierung bzw. eine Behandlungsmethode zur Verfügung zu stellen, die die beschriebenen Nachteile, insbesondere bei der Entfernung von kranker Nagelsubstanz der Fuß- oder Fingernägel, nicht aufweist.

Es wurde nun gefunden, dass man krankhaft veränderte Nagelareale an Fingern und Zehen, die durch Pilz befall entstehen können, einfach und effektiv ablösen und behandeln kann, wenn man die erfindungsgemäßen Zubereitungen auf die erkrankten Nägel aufträgt. Es handelt sich dabei um nicht feste und bevorzugt um nicht pastenartige Zubereitungen. Die erfindungsgemäßen, bevorzugt flüssigen, Zubereitungen lassen sich aufgrund ihrer Eigenschaften wie ein Nagellack leicht und gezielt mit einem Pinsel auftragen und sind nach ihrem Trocknen wisch- und abriebfest. Ein Abdecken der Zubereitungen mit Pflasterverbänden und das Auftragen eines speziellen Schutzfilmes auf die den Nagel umgebenden Hautflächen sowie das tägliche Baden der betroffenen Areale, z.B.Finger und Zehen, ist dabei nicht erforderlich. Ferner kommt es nicht zu einer unangenehmen Geruchsentwicklung bei der Behandlung.

Die Erfindung betrifft daher kosmetische und/oder pharmazeutische Zubereitungen, bevorzugt enthaltend einen hydrophilen Filmbildner, Wasser, Harnstoff, und mindestens eine antimykotisch wirksame Substanz und deren Verwendung zur Behandlung von pilzbefallenen Nagelareulen.

Die Erfindung betrifft ferner die Verwendung der erfindungsgemäßen Zubereitungen zur atraumatischen Nagelentfernung, d.h. zur Entfernung der krankhaft veränderten Nagelsubstanz. Gesundes Nagelmaterial wird dabei nicht angegriffen.

Die erfindungsgemäßen Zubereitungen enthalten Harnstoff bevorzugt in einer Menge von 70 bis 90 Gewichtsprozent, vorzugsweise 75 bis 85 Gewichtsprozent, bezogen auf die nichtflüchtigen Bestandteile der Zubereitungen. Auch mit anderen Hamstoffkonzentrationen kann der erfindungsgemäße Zweck, das Ablösen von infizierten Arealen, wie z.B. Finger- und Fußnägel oder von Hautareaten, die verhornt sein können, erreicht werden. Entsprechende Zubereitungen sind ebenfalls von der vorliegenden Erfindung umfasst. Veränderte Konzentrationen können dabei Einfluss auf die Behandlungsdauer haben.

Als hydrophile Filmbildner kommen beispielsweise Acryl- / Methacrylsäureester Copolymere, Polyvinylpyrrolidone, Polyvinylalkohole, Vinylacetat / Vinylpyrrolidon Copolymere, Vinylacetat / Crotonsäure Copolymere, Methylvinylether / Maleinsäure Copolymere, Carboxymethylcellulose, Hydroxyethylcellulose. Hydroxypropylcellulose und Hydroxypropylmethylcellulose in Frage. Besonders geeignet sind Potyvinylpyrrolidone. Sie werden in Mengen von bevorzugt 10 bis 30 Gewichtsprozent, vorzugsweise 15 bis 25 Gewichtsprozent, bezogen auf die nichtflüchtigen Bestandteile, eingesetzt.

Als weitere Hilfsmittel sind Weichmacher wie Glycerintriacetat oder 1.2-Propylenglycol, und Mittel zur Einstellung des pH-Wertes der Zubereitungen, zum Beispiel Milchsäureoder Zitronensäure, geeignet. Die pH-Wert regulierenden Substanzen werden bevorzugt in Mengen von 0,5 bis 5 Gewichtsprozent, bezogen auf die fertige Zubereitung, eingesetzt. Die Menge an Weichmachern liegt bevorzugt bei 1 bis 10 Gewichtsprozent, bezogen auf die fertige Zubereitung.

Die erfindungsgemäßen Zubereitungen können weiterhin in Kosmetika gebräuchliche Zusätze erhalten wie z.B. Weichmacher auf Phthalat- Glycerintriacetat- oder Campherbasis, Farbstoffe oder Farbpigmente, Perlglanzmittel, Sedimentationsverzögerer, Sulfonamidharze, Silikate, Riechstoffe, Netzmittel wie z.B. Natriumdioctylsulfo-succinat, Lanolinderivate, Lichtschutzmittel wie 2-Hydroxy-4-methoxybenzo-phenon oder antibakteriell wirksame Substanzen Gefärbte oder pigmentierte Nagellacke haben beispielsweise den Vorteil, dass die erfindungsgemäße Zubereitung dem Schönheitsempfinden des Patienten angepaßt werden kann und die derzeit bestehenden Nagelveränderungen für Dritte nicht unmittelbar sichtbar sind.

Ferner enthalten die Zubereitungen antimykotisch wirksame Substanzen wie z.B. Hydroxypyridone wie Ciclopirox, Ciclopiroxolamin, Piroctone oder Rilopirox, Morpholinderivate wie Amorolfinhydrochlorid oder Amorolfine, Azole wie Bifonazol, Butoconazole, Fluconazol, Clotrimazol, Econazol, Itraconazol, Miconazol, Omoconazole, Oxiconazol, Croconazol, Fenticonazol, Sulconazole, Tioconazol, Terconazole, Ketoconazol oder Isoconazol oder Allylverbindungen wie Terbinafinhydrochlorid, Terbinafin oder Naftifin sowie Griseofulvin, Haloprogin, Mepartricin, Undecylensäure, Dodecyltriphenylphosphoniumhydrochlorid, Lauroyloxypropylaminobuttersäure, Tolciclate, Tolnaftat und Butenafin enthalten.

Als geeignete Hydroxypyridone seien beispielsweise ferner genannt: 1-Hydroxy-4-methyl-6-n-hexyl-6-iso-hexyl-, -6-n-heptyl- oder -6-iso-heptyl-2-pyridon, 1-Hydroxy-4-methyl-6-octyl- oder -6-iso-octyl-2-pyridon, insbesondere das 1-Hydroxy-4-methyl-6-(2,4,4-trimethylpentyl)-2-pyridon, 1-Hydroxy-4-methyl-6-cyclohexyl-2-pyridon, 1-Hydroxy-4-methyl-6-cyclohexylmethyl- oder -6-cyclohexylethyl-2-pyridon, wobei der Cyclohexylrest jeweils auch einen Methylrest tragen kann, 1-Hydroxy-4-methyl-6-(2-bicyclo[2.2.1]heptyl)-2-pyridon, 1-Hydroxy-3,4-dimethyl-6-benzyl- oder -6-dimethylbenzyl-2-pyridon und 1-Hydroxy-4-methyl-6-(β-phenyl-ethyl)-2-pyridon.

Die genannten Wirkstoffe können auch in Form ihrer Salze oder Addukte, z.B. als Olamine, die für pharmakologische und/oder kosmetische Zwecke geeignet sind, verwendet werden.

Weiterhin werden von der vorliegenden Erfindung Zubereitungen umfasst, die anstelle oder in Kombination mit Harnstoff Alkalijodide, bevorzugt KJ oder NaJ, enthalten. Es können auch Mischungen der Alkalijodide verwendet werden. Die Konzentrationen (Gew.-%) der Alkalijodide bzw. der Harnstoff/Alkalijodid-Kombinationen entsprechen bevorzugt denjenigen, die für die harnstoffhaltigen Zubereitungen beschrieben wurden. Wasser als flüchtiger Bestandteil kann ganz oder teilweise durch andere Substanzen (Lösungsmittel) ersetzt werden. Voraussetzung ist, dass der Harnstoff bzw. die Alkalijodide in der verwendeten Substanz (Flüssigkeit) oder in dem verwendeten Substanzgernisch (Flüssigkeitsgemisch) ausreichend löslich sind. Anwendbar sind z.B. Wasser/Alkohol-Gemische. Der vollständige oder teilweise Ersatz von Wasser kann z.B. vorteilhaft zu einer schnelleren Ausbildung (Trocknung) des harnstoffhaltigen Films auf den behandelten Arealen führen.
Die erfindungsgemäßen Zubereitungen eignen sich außer für eine atraumatische Entfernung von krankhaft veränderter Nagelsubstanz allgemein zur Entfernung von krankhaft veränderten Keratinarealen, wie z.B. verhornten Hautarealen, die z.B. durch Pilz-, Bakterien- oder Virenbefall oder z.B. durch Psoriasis entstehen können. Eine solche Entfernung von krankhaft veränderten Keratinarealen kann zur Vorbereitung einer möglichst effizienten Folgebehandlung einer Person dienen.Die Herstellung der Zubereitungen erfolgt in an sich bekannter Weise durch Zusammengeben und Mischen der einzelnen Komponenten und einer - soweit erforderlich - der jeweiligen Zubereitung angepassten Weiterverarbeitung.

Die vorliegende Erfindung wird durch die folgenden Beispiele näher erläutert, jedoch nicht auf diese beschränkt. Soweit nichts anderes vermerkt, sind die Mengenangaben (Gew.-%) auf das Gewicht der fertigen Zubereitung (einschließlich der flüchtigen Bestandteile; z.B. Wasser) bezogen.

### Beispiel 1

Eine nagelablösende Zubereitung weist folgende Zusammensetzung auf:

| | |
|---|---|
| Harnstoff | 40,0% |
| Polyvinylpyrrolidon (Molekulargewicht etwa 11 500) | 10,0% |
| Demineralisiertes Wasser | 50,0% |

### Beispiel 2

Eine nagelablösende Zubereitung weist folgende Zusammensetzung auf:

| | |
|---|---|
| Harnstoff | 40,0% |
| Polyvinylpyrrolidon (Molekulargewicht etwa 11 500) | 10,0% |
| Glycerintriacetat | 4,0% |
| Milchsäure | 1,0% |
| Demineralisiertes Wasser | 45,0% |

### Beispiel 3

Eine nagelablösende Zubereitung weist folgende Zusammensetzung auf:

| | |
|---|---|
| Harnstoff | 40,0% |
| Vinylacetat / Vinylpyrrolidon Copolymer | 7,5% |
| Glycerintriacetat | 2,5% |
| Demineralisiertes Wasser | 50,0% |

### Beispiel 4

Eine nagelablösende-Zubereitung weist folgende Zusammensetzung auf:

| | |
|---|---|
| Harnstoff | 40,0% |
| Polyvinylalkohol | 8,0% |
| 1.2-Propylenglycol | 2,0% |
| Demineralisiertes Wasser | 50,0% |
| Beispiel 5 | |

Eine erfindungsgemäße Zubereitung weist folgende Zusammensetzung auf:

| | |
|---|---|
| Harnstoff | 40,0% |
| Polyvinylpyrrolidon (Molekulargewicht etwa 25 000) | 10,0% |
| Fluconazol | 2,5% |
| Milchsäure | 1,0% |
| Demineralisiertes Wasser | 46,5% |

### Beispiel 6

Eine nagelablösende Zubereitung weist folgende Zusammensetzung auf:

| | |
|---|---|
| Harnstoff | 40% |
| Polyvinylpyrrolidon | 10% |
| Milchsäure | 1% |
| Wasser | 49% |

### Vergleichsbeispiel

Eine Pastenzubereitung aus dem Stand der Technik weist folgende Zusammenseteung auf:

| | |
|---|---|
| Harnstoff | 40,0% |
| Wasserfreies Wollfett | 20,0% |
| Gebleichtes Wachs | 5,0% |
| Weiße Vaseline | 35,0% |

### Wirksamkeitsprüfung

Je 20 an Nagelpilz erkrankte Patienten wurden mit den genannten Zubereitungen bzw. mit einer Paste aus dem Stand der Technik, gemäß Vergleichsbeispiel, behandelt.

Die Zubereitungen wurden einmal täglich vor dem Schlafengehen gezielt mit einem Pinsel auf die befallenen Nägel aufgetragen. Der etwa 2 Minuten nach dem Auftragen auf den Nägeln entstandene harnstoffhaltige Film war wisch- und wasserfest. Ein besonderer Schutz der die Nägel umgebenden Hautflächen sowie das Anbringen von Pflasterverbänden war daher nicht erforderlich. Aufgrund des hohen Wassergehaltes der Zubereitungen wurden die befallenen Finger- bzw. Zehennägel nicht zusätzlich gebadet.

Die Hamstoffpaste, gemäß Vergleichsbeispiel, wurde ebenfalls einmal täglich aufgetragen, wobei die Nägel umgebenden Hautflächen mit einer Schutzpaste abgedeckt wurden. Die betroffenen Finger- oder Zehennägel wurden für jeweils 24 Stunden mit einem Pflasterverband zugeklebt und nach dem Entfernen des Verbandes etwa 10 Minuten in warmen Wasser gebadet.

### Ergebnis:

Nach etwa 7 Tagen Behandlung konnten die befallenen Nagelareale und die subungualen Gewebstrümmer von beiden Patientengruppen leicht entfernt werden. Die Behandlung mit den erfindungsgemäßen Zubereitungen wurde jedoch aufgrund ihrer vereinfachten Handhabung ― und der damit für den Patienten verbundenen Zeitersparnis ― stark bevorzugt angenommen.

## Patentansprüche

1. Verwendung von Wasser, Harnstoff, einem Filmbildner und mindestens einer antimykotisch wirksamen Substanz zur Herstellung einer pharmazeutischen Zubereitung zur Entfernung und Behandlung von pilzbefallenen Nagelarealen.

2. Verwendung nach Anspruch 1 zur gleichzeitigen Behandlung von Nagelmykosen.

3. Verwendung nach Anspruch 1 oder 2, **dadurch gekennzeichnet, dass** die Zubereitung Harnstoff in einer Menge von 70 bis 90 Gewichtsprozent enthält, bezogen auf die nichtflüchtigen Bestandteile der Zubereitung.

4. Verwendung nach einem oder mehreren der Ansprüche 1 bis 3, **dadurch gekennzeichnet, dass** die Zubereitung den Filmbildner in einer Menge von 10 bis 30 Gewichtsprozent enthält, bezogen auf die nichtflüchtigen Bestandteile der Zubereitung.

5. Verwendung nach einem oder mehreren der Ansprüche 1 bis 4; **dadurch gekennzeichnet, dass** die Zubereitung als Filmbildner Polyvinylpyrrolidon enthält.

6. Verwendung nach einem oder mehreren der Ansprüche 1 bis 5, **dadurch gekennzeichnet, dass** die Zubereitung einen Weichmacher und/oder pH-Wert regulierende Substanzen enthält.

7. Verwendung nach einem oder mehreren der Ansprüche 1 bis 6, **dadurch gekennzeichnet, dass** die Zubereitung eine antimykotisch wirksame Substanz aus der Gruppe der Hydroxpyridone wie Ciclopirox, Ciclopiroxolamin, Piroctone oder Rilopirox, Morpholinderivate wie Amorolfinhydrochlorid oder Amorolfine, Azole wie Bifonazol, Fluconazol, Clotrimazol, Econazol, Itraconazol, Miconazol, Oxiconazol, Croconazol, Fenticonazol, Tioconazol, Ketoconazol oder Isoconazol oder Allylverbindungen wie Terbinafinhydrochlorid, Terbinafin oder Naftifin sowie Griseofulvin, Haloprogin, Mepartricin, Undecylensäure, Dodecyltriphenylphosphoniumhydrochlorid, Lauroyloxypropylaminobuttersäure, Tolciclate, Tolnaftat und Butenafin oder deren für pharmakologische Zwecke geeigneten Salze oder Addukte enthält.

8. Verwendung nach einem oder mehreren der Ansprüche 1 bis 7, **dadurch gekennzeichnet, dass** die Zubereitung als antimykotisch wirksame Substanz Fluconazol enthält.

9. Verwendung nach einem oder mehreren der Ansprüche 1 bis 8, **dadurch gekennzeichnet, dass** in der Zubereitung Harnstoff ganz oder teilweise durch Alkalijodide ersetzt ist.

10. Verwendung nach einem oder mehreren der Ansprüche 1 bis 9, **dadurch gekennzeichnet, dass** in der Zubereitung Wasser ganz oder teilweise durch Substanzen ersetzt ist, in denen oder in deren Mischung mit Wasser Harnstoff und/oder Alkalijodide löslich sind.

11. Verwendung nach einem oder mehreren der Ansprüche 1 bis 10, **dadurch gekennzeichnet, dass** es sich um eine flüssige Zubereitung handelt.

12. Verwendung nach einem oder mehreren der Ansprüche 1 bis 11, **dadurch gekennzeichnet, dass** es sich bei der Zubereitung um einen Nagellack handelt.

13. Pharmazeutische Zubereitung enthaltend Wasset, Fluconazol, 70 bis 90 Gewichtsprozent Harnstoff und 10 bis 30 Gewichtsprozent Filmbildner, jeweils bezogen auf die nichtflüchtigen Bestandteile der Zubereitung.

## Claims

1. The use of water, urea, a film former and at least one substance with antimycotic activity for producing a pharmaceutical preparation for the removal and treatment of fungus-infected areas of nails.

2. The use as claimed in claim 1 for simultaneous treatment of nail mycoses.

3. The use as claimed in claim 1 or 2, wherein the preparation comprises urea in an amount of from 70 to 90 percent by weight based on the involatile ingredients of the preparation.

4. The use as claimed in one or more of claims 1 to 3, wherein the preparation comprises the film former in an amount of from 10 to 30 percent by weight, based on the involatile ingredients of the preparation.

5. The use as claimed in one or more of claims 1 to 4, wherein the preparation contains polyvinylpyrrolidone as film former.

6. The use as claimed in one or more of claims 1 to 5, wherein the preparation comprises a plasticizer and/or pH-regulating substances.

7. The use as claimed in one or more of claims 1 to 6, wherein the preparation comprises a substance with antimycotic activity from the group of hydroxypyridones such as ciclopirox, ciclopiroxolamine, piroctone or rilopirox, morpholine derivatives such as amorolfine hydrochloride or amorolfine, azoles such as bifonazole, fluconazole, clotrimazole, econazole, itraconazole, miconazole, oxiconazole, croconazole, fenticonazole, tioconazole, ketoconazole or isoconazole or allyl compounds such as terbinafine hydrochloride, terbinafine or naftifine, and griseofulvin, haloprogin, mepartricin, undecylenic acid, dodecyltriphenylphosphonium hydrochloride, lauroyloxypropylaminobutyric acid, tolciclate, tolnaftate and butenafine or their salts or adducts suitable for pharmacological purposes.

8. The use as claimed in one or more of claims 1 to 7, wherein the preparation comprises fluconazole as substance with antimycotic activity.

9. The use as claimed in one or more of claims 1 to 8, wherein urea is wholly or partly replaced by alkali metal iodides in the preparation.

10. The use as claimed in one or more of claims 1 to 9, wherein water in the preparation is replaced wholly or partly by substances in which or in whose mixtures with water urea and/or alkali metal iodides are soluble.

11. The use as claimed in one or more of claims 1 to 10, wherein the preparation is liquid.

12. The use as claimed in one or more of claims 1 to 11, wherein the preparation is a nail varnish.

13. A pharmaceutical preparation comprising water, fluconazole, from 70 to 90 percent by weight of urea and from 10 to 30 percent by weight of film formers, in each case based on the involatile ingredients of the preparation.

## Revendications

1. Utilisation d'eau, d'urée, d'un agent filmogène et d'au moins une substance active antimycosique pour la fabrication d'une préparation pharmaceutique destinée à l'élimination et au traitement de zones des ongles attaquées par des champignons.

2. Utilisation selon la revendication 1 pour le traitement simultané de mycoses des ongles.

3. Utilisation selon la revendication 1 ou 2, **caractérisée en ce que** la préparation contient de l'urée à raison de 70 à 90 pour cent en poids par rapport aux composants non volatils de la préparation.

4. Utilisation selon l'une quelconque ou plusieurs des revendications 1 à 3, **caractérisée en ce que** la préparation contient l'agent filmogène à raison de 10 à 30 pour cent en poids par rapport aux composants non volatils de la préparation.

5. Utilisation selon l'une quelconque ou plusieurs des revendications 1 à 4, **caractérisée en ce que** la préparation contient, en tant qu'agent filmogène, de la polyvinylpyrrolidone.

6. Utilisation selon l'une quelconque ou plusieurs des revendications 1 à 5, **caractérisée en ce que** la préparation contient un plastifiant et/ou des substances régulant le pH.

7. Utilisation selon l'une quelconque ou plusieurs des revendications 1 à 6, **caractérisée en ce que** la préparation contient une substance active antimycosique issue du groupe des hydroxypyridones telles que le ciclopirox, la ciclopiroxolamine, la piroctone ou le rilopirox, des dérivés de morpholine tels que le chlorhydrate d'amorolfine ou l'amorolfine, des azoles tels que le bifonazole, le fluconazole, le clotrimazole, l'éconazole, l'itraconazole, le miconazole, l'oxiconazole, le croconazole, le fenticonazole, le tioconazole, le cétoconazole ou l'isoconazole, ou des composés allyliques tels que le chlorhydrate de terbinafine, la terbinafine ou la naftifine ainsi que la griséofulvine, l'haloprogine, la mépartricine, l'acide undécylénique, le chlorhydrate de dodécyltriphénylphosphonium, l'acide lauroyloxypropylaminobutyrique, le tolciclate, le tolnaftate et la buténafine, ou leurs sels ou produits d'addition appropriés sur le plan pharmacologique.

8. Utilisation selon l'une quelconque ou plusieurs des revendications 1 à 7, **caractérisée en ce que** la préparation contient, en tant que substance active antimycosique, du fluconazol.

9. Utilisation selon l'une quelconque ou plusieurs des revendications 1 à 8, **caractérisée en ce que** dans la préparation, l'urée est remplacée entièrement ou partiellement par des iodures alcalins.

10. Utilisation selon l'une quelconque ou plusieurs des revendications 1 à 9, **caractérisée en ce que** dans la préparation, l'eau est remplacée entièrement ou partiellement par des substances dans lesquelles, ou dans les mélanges aqueux desquelles, l'urée et/ou les iodures alcalins sont solubles.

11. Utilisation selon l'une quelconque ou plusieurs des revendications 1 à 10, **caractérisée en ce qu'**il s'agit d'une préparation liquide.

12. Utilisation selon l'une quelconque ou plusieurs des revendications 1 à 11, **caractérisée en ce** la préparation est un vernis à ongles.

13. Préparation pharmaceutique contenant de l'eau, du fluconazol, 70 à 90 pour cent en poids d'urée et 10 à 30 pour cent en poids d'agent filmogène, à chaque fois par rapport aux composants non volatils de la préparation.
